# EUROPÄISCHE PATENTSCHRIFT

(11) **EP 2 919 838 B1**
(45) Veröffentlichungstag und Bekanntmachung des Hinweises auf die Patenterteilung: **24.07.2019**
(21) Anmeldenummer: 13794841.0
(22) Anmeldetag: 12.11.2013
(51) Int. Cl.: A61M 5/34, A61M 5/24

(54) **AUFSATZ FÜR EINE SPRITZE ODER KARPULE**
ATTACHMENT FOR A SYRINGE OR CARTRIDGE
CONNEXION POUR SERINGUE OU CARTOUCHE

(30) Priorität: 15.11.2012 DE 102012022359
(43) Veröffentlichungstag der Anmeldung: 23.09.2015
(73) Patentinhaber: Vetter Pharma-Fertigung GmbH & Co. KG, 88212 Ravensburg (DE)
(72) Erfinder: HUND, Petra, 88276 Berg (DE); ZENKER, Jochen, 88212 Ravensburg (DE)
(74) Vertreter: Kordel, Mattias
(86) Internationale Anmeldenummer: PCT/EP2013/073621
(87) Internationale Veröffentlichungsnummer: WO 2014/076081

(56) Entgegenhaltungen:
- EP-A1- 1 099 450
- EP-A1- 1 502 616
- DE-A1-102009 007 250
- DE-A1-102011 013 791

## Beschreibung

Die Erfindung betrifft einen Aufsatz für eine Spritze, eine Karpule oder dergleichen gemäß Oberbegriff des Anspruchs 1, sowie eine Spritze, eine Karpule oder dergleichen mit den Merkmalen des Oberbegriffs des Anspruchs 10.

Aufsätze für Spritzen, Karpulen oder dergleichen sind bekannt (EP 1 502 616 A1; EP 1 099 450 A1; DE 10 2009 007 250 A1; DE 10 2011 013 791 A1). Sie werden auch als Adapter verwendet und dienen dazu, ein Befestigungsmittel für eine Kanüle oder eine sonstige Vorrichtung, beispielsweise eine Injektionseinrichtung oder dergleichen bereitzustellen. Beispielsweise sind derartige Aufsätze, die auf einen Fortsatz der Spritze, Karpule oder dergleichen aufgesetzt werden, in der Regel mit einem Innengewinde versehen, in welches ein Einsatz der Kanüle eingeschraubt werden kann. Häufig sind Aufsätze der hier angesprochenen Art auch als Sicherheits- oder Garantieverschluss ausgebildet und weisen ein erstes Befestigungsteil auf, welches fest auf einen endständigen Fortsatz einer Spritze, einer Karpule oder dergleichen aufgesetzt wird und dort verriegelnd gehalten wird. Über eine Sollbruchlinie ist mit dem Befestigungskörper bei derartigen Verschlüssen eine Kappe verbunden, welche das freie Ende des Fortsatzes und damit den Innenraum der Spritze, Karpule oder dergleichen sicher verschließt. Wird die Kappe abgenommen, reißt die Sollbruchlinie auf, sodass irreversibel erkennbar ist, dass die Kappe entfernt wurde. Damit wird auch ein Schutz vor Manipulationen an dem Verschluss gewährleistet. Der Fortsatz weist ein freies Ende auf, außerdem in einem Abstand zu diesem mindestens eine in die Außenfläche des Fortsatzes eingebrachte Vertiefung. Vorzugsweise wird hier eine Ringnut vorgesehen, in welche der auf den Fortsatz aufgesetzte Aufsatz verriegelnd eingreift. Häufig wird eine Spritze, eine Karpule oder dergleichen nach dem Aufbringen des Aufsatzes einem Sterilisationsverfahren unterworfen, bei welchem beispielsweise Heißdampf eingesetzt wird. Die beim Sterilisieren verwendeten hohen Temperaturen beeinflussen das Material des Aufsatzes, welches unter Vorspannung an dem Fortsatz eingreift. Es ist daher möglich, dass das Material des Aufsatzes so beeinträchtigt wird oder gar versprödet, dass ein sicherer Halt des Aufsatzes auf dem Fortsatz nicht mehr gewährleistet werden kann. Ein Nachteil ist auch, dass Aufsätze der hier angesprochenen Art, selbst wenn sie nicht einem Sterilisationsvorgang unter Wärmeeinfluss unterzogen werden, nicht immer sicher genug an dem Fortsatz gehalten werden, sodass beim Transport oder bei der Handhabung der Spritze, Karpule oder dergleichen der Aufsatz versehentlich gelöst wird. Dies ist insbesondere dann unerwünscht, wenn der Aufsatz als Originalitätsverschluss ausgebildet ist und den Innenraum der Spritze, Karpule oder dergleichen steril verschließen soll. Auch hat es sich gezeigt, dass ein Verdrehen des Aufsatzes auf dem Fortsatz häufig unerwünscht ist.

Aufgabe der Erfindung ist es daher, einen Aufsatz für eine Spritze, Karpule oder dergleichen zu schaffen, wobei die oben genannten Nachteile vermieden werden.

Zur Lösung dieser Aufgabe wird ein Aufsatz vorgeschlagen, welcher die in Anspruch 1 genannten Merkmale umfasst. Dieser Aufsatz ist für eine Spritze, eine Karpule oder dergleichen vorgesehen, die ein distales Ende aufweist, also ein Ende, welches bei Benutzung dem Patienten zugewandt ist. An diesem ist ein endständiger Fortsatz vorgesehen, der eine Außenfläche, ein freies Ende und mindestens eine in einem Abstand zu diesem Ende in die Außenfläche eingebrachte Vertiefung aufweist.

Der hier angesprochen Aufsatz ist nicht nur für eine Spritze oder eine Karpule verwendbar, sondern beispielsweise auch für einen Pen, Adapter für Schläuche, Injektionseinrichtungen oder dergleichen. Wesentlich ist, dass der hier angesprochene Aufsatz mit einem Fortsatz der oben angesprochenen Art zusammenwirkt und so ausgelegt ist, dass eine sichere Verbindung zwischen dem Aufsatz und dem Fortsatz gewährleistet ist. Im Folgenden wird beispielhaft von einem Fortsatz an einer Spritze oder Karpule ausgegangen, obwohl, wie dargelegt, der Fortsatz auch an anderen insbesondere medizinischen Einrichtungen vorgesehen sein kann.

Der Aufsatz weist einen ringförmigen Grundkörper auf und ist damit auf den Fortsatz aufbringbar, wobei der Grundkörper einen Freiraum umschließt, in den der Fortsatz einführbar ist. Der Grundkörper umfasst zwei Materialien, von denen ein erstes Material formstabil und ein zweites Material weicher ist als das formstabile Material und verformbar ist. Das erste Material dient dazu, den Aufsatz gut handhabbar zu machen. Mit formstabilem Material ist also gemeint, dass der Aufsatz auch unter Aufwendung einer gewissen Kraft auf den Fortsatz einer Spritze, einer Karpule oder dergleichen aufgesetzt werden kann, ohne bleibend verformt oder gar zerstört zu werden. Auch bei einer Benutzung der Spritze oder Karpule, bei Transport und bei der Handhabung des Aufsatzes vor und bei der Benutzung der Spritze oder Karpule muss der Aufsatz so stabil sein, dass er seine Form behält und damit sicher an dem Fortsatz gehalten wird. Das zweite Material ist weicher und verformbar, sodass es beim Aufbringen des Aufsatzes auf den Fortsatz eine Innenkontur annimmt, die der Außenkontur des Fortsatzes entspricht. Der Grundkörper soll an dem Fortsatz sicheren Halt finden und weist daher einen Eingriffsbereich auf, der in auf den Fortsatz aufgebrachtem Zustand in mindestens eine Vertiefung desselben eingreift. Der Aufsatz zeichnet sich dadurch aus, dass dessen Grundkörper entlang einer gedachten Umfangslinie erste Bereiche mit dem ersten Material und zweite Bereiche mit dem zweiten Material aufweist, wobei das zweite Material in auf den Fortsatz aufgebrachtem Zustand in die mindestens eine Vertiefung eingeformt, in anderen Worten mit dieser verpresst wird, und wobei das zweite Material in das erste Material eingebettet ist. Die gedachte Umfangslinie befindet sich im aufgebrachtem Zustand im Bereich der mindestens einen Vertiefung des Fortsatzes, sodass durch das Zusammenspiel der beiden Bereiche, die das erste und zweite Material umfassen, ein besonders sicherer Halt am Fortsatz der Spritze oder Karpule gewährleistet ist. Die Einformung des zweiten, verformbaren Materials in die mindestens eine Vertiefung ist optimal sichergestellt, weil das zweite weichere Material in dem formstabilen Material untergebracht ist. Dies führt dazu, dass das verformbare Material nicht ausweichen kann und in der mindestens einen Vertiefung in der Außenfläche des Fortsatzes sicheren Halt findet. Dies führt dazu, dass der Aufsatz sehr sicher gehalten wird und nicht nur bei axial wirkenden Kräften, die in Längsrichtung der Spritze oder Karpule beziehungsweise in Richtung einer Längsachse des Aufsatzes wirken, sicher gehalten wird. Das in die mindestens eine Vertiefung eingeformte verformbare Material stellt darüber hinaus sicher, dass der Aufsatz verdrehsicher auf dem Fortsatz gehalten ist und bei einem bei Gebrauch des Aufsatzes von außen einwirkenden Drehmoment keine Relativdrehung gegenüber dem Fortsatz durchführt.

Erfindungsgemäß weist der Aufsatz am Ende seines Grundkörpers einen Ringkörper auf, der in auf den Fortsatz aufgebrachtem Zustand einen ringförmigen Bereich des Fortsatzes umgreift, wobei in diesem Bereich des Fortsatzes die mindestens eine Vertiefung angeordnet ist, wobei der Ringkörper in Umfangsrichtung gesehen vorzugsweise in gleichem Abstand zueinander angeordnete Segmente umfasst, welche die ersten Bereiche bilden und daher aus dem ersten Material bestehen. In den Zwischenräumen zwischen diesen Segmenten sind auch als Taschen bezeichnete Ausnehmungen vorgesehen, die das zweite Material aufnehmen und die zweiten Bereiche bilden.

Ein besonders bevorzugtes Ausführungsbeispiel des Fortsatzes zeichnet sich dadurch aus, dass dieser in einem Zwei-Komponenten-Spritzgussverfahren herstellbar ist, wobei das oben genannte erste Material und auch das in Anspruch 1 aufgeführte zweite Material bei diesem Spritzgussverfahren verwendet werden. Dieses Herstellungsverfahren ermöglicht es, das zweite Material einfach und kostengünstig in das erste Material einzubetten. Überdies ist es möglich, gewünschte Konturen, auch Hinterschnitte, auf einfache und kostengünstige Weise zu realisieren. Die relativ freie Formgestaltung bei der Herstellung des Aufsatzes ermöglicht die Realisierung einer hohen Funktionssicherheit.

Mindestens ein Segment weist bevorzugt wenigstens einen vorzugsweise ringförmig ausgebildeten Hohlraum auf, der leer sein kann, vorzugsweise aber das zweite Material enthält. Der Hohlraum wird durch einen kreisbogenförmigen Wandabschnitt des Segments gegenüber dem Hohlraum abgegrenzt. Zwei von dem Wandabschnitt ausgehende Wandbereiche begrenzen den Hohlraum seitlich, wobei der Wandabschnitt und die Wandbereiche nachgiebig ausgebildet sind. Dadurch ergibt sich Folgendes: Wenn der Aufsatz auf einen Fortsatz aufgesteckt wird, wirken radial nach außen gerichtete Kräfte, also vom Freiraum her, auf den Wandabschnitt, die diesen radial nach außen drücken. Dadurch werden die Wandbereiche so verformt, dass sie seitlich nach außen gedrängt werden; dies insbesondere dann, wenn der Hohlraum mit dem zweiten Material gefüllt ist und eine Verlagerung der Wandbereiche nach innen verhindert. Durch die Auswärtsbewegung der seitlichen Wandbereiche werden die an ein Segment seitlich, das heißt in Umfangsrichtung angrenzenden Ausnehmungen schmaler. In diesen Ausnehmungen vorhandenes zweites Material wird dadurch aus den Ausnehmungen herausgepresst und formt sich daher besonders gut in die mindestens eine Vertiefung des Fortsatzes ein.

Diese Ausgestaltung sorgt also für einen besonders sicheren Halt des Aufsatzes an einer Spritze, Karpule oder dergleichen, wobei der Aufsatz gegen axiale aber auch gegen in Umfangsrichtung wirkende Kräfte sicher lagefest gehalten wird.

Weitere Ausführungsbeispiele des Aufsatzes ergeben sich aus den Unteransprüchen.

Aufgabe der Erfindung ist es außerdem, eine Spritze, eine Karpule oder dergleichen zu schaffen, welche die oben genannten Nachteile nicht aufweist.

Zur Lösung dieser Aufgabe wird eine Spritze, eine Karpule oder dergleichen vorgeschlagen, welche die in Anspruch 10 aufgeführten Merkmale, insbesondere einen Aufsatz der oben definierten Art umfasst.

Die Erfindung wird im Folgenden anhand der Zeichnung näher erläutert. Es zeigen:
- Figur 1: eine Draufsicht auf eine Stirnseite eines ersten Ausführungsbeispiels eines Aufsatzes;
- Figur 2: eine perspektivische Ansicht eines in Längsrichtung aufgeschnittenen zweiten Ausführungsbeispiels eines Aufsatzes;
- Figur 3: eine Draufsicht eine Stirnseite eines dritten Ausführungsbeispiels eines Aufsatzes;
- Figur 4: eine perspektivische Ansicht des aufgeschnittenen zweiten Ausführungsbeispiels gemäß Figur 3;
- Figur 5: eine Schnittdarstellung des auf einen Fortsatz einer Spritze oder Karpule aufgesteckten dritten Ausführungsbeispiels des Aufsatzes;
- Figur 6: eine perspektivische Ansicht eines in Längsrichtung aufgeschnittenen vierten Ausführungsbeispiels eines Aufsatzes und
- Figur 7: eine perspektivische Ansicht des in einer anderen Ebene in Längsrichtung aufgeschnittenen vierten Ausführungsbeispiels des Aufsatzes.

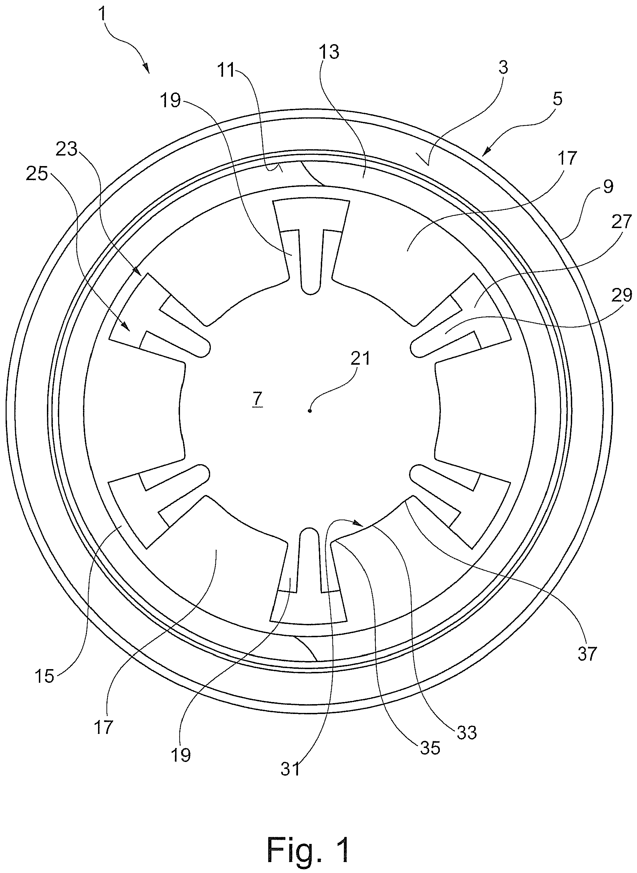

Figur 1 zeigt in Draufsicht ein erstes Ausführungsbeispiel eines Aufsatzes 1, und zwar auf dessen Stirnseite 3. Der hier dargestellte Aufsatz 1 ist so ausgebildet, dass er auf eine hier nicht dargestellte Spritze, Karpule oder dergleichen aufsetzbar ist, nämlich auf einen endständigen Fortsatz an einem distalen Ende der Spritze, Karpule oder dergleichen. Ist der Fortsatz an einer Spritze oder Karpule vorgesehen, so weist dieser einen auch als Spritzen- beziehungsweise Karpulenzylinder bezeichneten Körper mit einem Innenraum zur Aufnahme einer Substanz auf. Dabei kann der Innenraum einen oder mehrere Substanzen aufnehmen, die einem Patienten verabreicht werden sollen, oder aber auch dazu dienen, Flüssigkeiten, insbesondere von Patienten, aufzunehmen, die einer medizinischen Untersuchung oder Behandlung unterworfen werden sollen. Der Körper weist ein distales Ende auf, an welchem der Fortsatz vorgesehen ist. Es sei hier noch mal darauf hingewiesen, dass ein derartiger Fortsatz auch bei Pens, Adaptern für Schläuche oder medizinische Geräte vorgesehen sein kann, und dass hier lediglich beispielhaft auf Fortsätze an einem Ende von Spritzen oder Karpulen ausgegangen wird.

Der Aufsatz 1 weist einen Grundkörper 5 auf, der einen Freiraum 7 einschließt. Der Grundkörper 5 ist vorzugsweise zylindrisch ausgebildet. Der Freiraum 7 ist im Wesentlichen kreisrund ausgebildet, damit er den Fortsatz einer Spritze, einer Karpule oder dergleichen aufnehmen kann. Der Grundkörper 5 weist einen Hülsenabschnitt 9 auf, der den Fortsatz umgreift und vorzugsweise auf seiner Innenfläche 11 mit einem Gewinde 13 versehen ist. An den Hülsenabschnitt 9 schließt sich in einem Abstand zur Stirnseite 3, nämlich am gegenüberliegenden Ende des Grundkörpers 5 ein Ringkörper 15 an, der den Fortsatz in einem auf den Fortsatz aufgesetzten oder aufgebrachten Zustand einen ringförmigen Bereich dieses Fortsatzes umgreift. Der Ringkörper 15 weist mindestens ein Segment 17 auf und wenigstens eine auch als Tasche bezeichnete Ausnehmung 19. Das mindestens eine Segment weist ein erstes Material und die mindestens eine Ausnehmung ein zweites Material auf, wobei das erste Material formstabil und das zweite Material weicher ist als das formstabile Material, außerdem verformbar.

Das hier dargestellte Ausführungsbeispiel des Aufsatzes 1 zeichnet sich gegenüber einer Grundversion des Aufsatzes mit mindestens einem Segment und mindestens einer Ausnehmung dadurch aus, dass - in Umfangsrichtung gesehen - vorzugsweise in gleichem Abstand zueinander angeordnete Segmente vorgesehen sind, hier sechs in gleichem Abstand zueinander angeordnete Segmente. Zwischen den Segmenten 17, die vorzugsweise identisch ausgebildet sind, befindet sich die mindestens eine Ausnehmung 19, wobei bei dem bevorzugten Ausführungsbeispiel sechs in gleichem Umfangsabstand zueinander angeordnete gleichartige Ausnehmungen 19 zwischen den Segmenten 17 vorgesehen sind.

Die Segmente 17 und die Ausnehmungen 19 sind vorzugsweise keilförmig ausgebildet und zwar derart, dass sie sich - in Richtung auf das Zentrum 21 des Freiraums 7 gesehen - verjüngen. Durch dieses Zentrum 21 verläuft senkrecht zur Bildebene von Figur 1 die hier nicht dargestellte Mittelachse des Aufsatzes 1.

Der Aufsatz 1 weist eine Haltevorrichtung 23 auf, die mindestens ein Halteelement 25 umfasst. Bei dem Ausführungsbeispiel des Aufsatzes mit einem Segment und einer Ausnehmung ist lediglich ein Halteelement vorgesehen. Bei dem hier dargestellten Ausführungsbeispiel mit sechs Segmenten 17 und sechs Ausnehmungen 19 umfasst die Haltevorrichtung 23 sechs Halteelemente 25, die jeweils in einer Ausnehmung 19 untergebracht, vorzugsweise dort eingebettet sind. Die Haltevorrichtung 23 dient dazu, den auf einen Fortsatz aufgesetzten Aufsatz 1 sicher zu halten, sodass er auch bei Einwirkung von in axialer Richtung beziehungsweise in Längsrichtung des Aufsatzes 1 eingreift oder aber in Umfangsrichtung wirkenden Kräften, also Drehmomenten, sich nicht von dem Fortsatz löst, sondern sicher gehalten wird.

Bei dem hier dargestellten Ausführungsbeispiel des Aufsatzes 1 ist vorgesehen, dass die Halteelemente 25 ein Grundelement 27 und einen davon ausgehenden Fortsatz 29 umfassen, wobei der Fortsatz 29 bei dem hier dargestellten Ausführungsbeispiel mittig von dem kreissegmentartig ausgebildeten Grundelement 27 ausgeht. Da das Halteelement 25 in einer sich in Richtung zu Zentrum 21 verjüngenden Ausnehmung 19 untergebracht ist, und das Grundelement 27 sich über die gesamte - in Umfangsrichtung gemessene - Breite der Ausnehmung 19 erstreckt, kann das Halteelement 25 nicht nach innen in den Freiraum 7 gelangen. Vorzugsweise ist das Halteelement 25 in die Ausnehmung 19 eingepresst.

Das mindestens eine Segment 17 weist eine dem Zentrum 21 zugewandte, hier also senkrecht zur Bildebene der Figur verlaufende Innenseite 31 auf. Der Abstand zweier diametral gegenüberliegenden Innenseiten 31 definiert einen Innenradius des Freiraums 7, der vorzugsweise kleiner oder gleich ist wie der Außendurchmesser eines ringförmigen Bereichs des hier nicht dargestellten Fortsatzes der Spritze, Karpule oder dergleichen, der im aufgesetzten Zustand des Aufsatzes 1 mit dessen Ringkörper 15 zusammenwirkt. Vorzugsweise ist der Innendurchmesser des Freiraums 7 auch deshalb kleiner als der Außendurchmesser des Ringbereichs des Fortsatzes, dass der Aufsatz 1 beim Aufbringen auf den Fortsatz aufgeweitet wird und sich schon aus diesem Grund durch Reibkräfte an der Außenfläche des Fortsatzes hält.

Die Innenseite 31 kann als ebene Fläche ausgebildet sein, auf deren Mittellinie eine vom Zentrum 21 ausgehende radiale Linie senkrecht steht.

Vorzugsweise ist der mittlere Bereich 33 kreisbogenförmig ausgebildet und ist auf einer gedachten Kreislinie angeordnet, deren Mittelpunkt im Zentrum 21 liegt. An den mittleren Bereich 33 schließen sich - in Umfangsrichtung gesehen - vorzugsweise außen Seitenbereiche 35 und 37 an, die ausgehend von dem mittleren Bereich 33 nach außen abfallen, also einen zunehmenden Abstand zum Zentrum 21 aufweisen.

Die dem Zentrum 21 zugewandten Enden der Fortsätze 29 der Halteelemente 25 liegen in einem - entlang einer gedachten Durchmesserlinie - gemessenen Abstand zueinander, der kleiner ist als der Abstand zwischen den mittleren Bereichen 33 gegenüberliegender Segmente 17.

Das erste Ausführungsbeispiel des Aufsatzes 1 weist also mindestens ein Segment 17 und mindestens eine Ausnehmung 19 mit jeweils einem Halteelement 25 einer Haltevorrichtung 23 auf, vorzugsweise, wie in Figur 1 dargestellt, sechs in Umfangsrichtung verteilte Segmente 17, sechs zwischen den Segmenten 17 angeordnete Ausnehmungen 19 mit sechs Halteelementen 25. Wird ein Aufsatz 1 der hier beschriebenen Art auf einen Fortsatz einer Spritze, Karpule oder dergleichen aufgesteckt, so liegt der Ringkörper 15 im Bereich der Ringfläche des Fortsatzes. Der Ringkörper 15 weist - in Umfangsrichtung gesehen - mindestens einen ersten Bereich auf, der von der Innenseite 31 eines Segments 17 gebildet wird, und mindestens einen zweiten Bereich, der durch das Halteelement 25, hier durch dessen Fortsatz 29, definiert ist. Besonders bevorzugt sind mehrere Segmente 17 und mehrere Ausnehmungen 19 vorgesehen, die sich in Umfangsrichtung abwechseln.

Der Aufsatz 1 weist in seinem mindestens einen ersten Bereich ein erstes formstabiles Material auf, und in seinem mindestens einen zweiten Bereich ein zweites Material auf, welches weicher ist als das formstabile Material und verformbar. Wird also ein Aufsatz 1 dieser Art auf einen Fortsatz aufgebracht, so wird das zweite weiche Material des mindestens einen, vorzugsweise der sechs zweiten Bereiche in mindestens eine Vertiefung in der Außenfläche des Fortsatzes eingeformt beziehungsweise eingepresst. Dabei ist vorgesehen, dass die Anzahl der Vertiefungen in der Außenfläche des Fortsatzes vorzugsweise abgestimmt ist auf die Anzahl der zweiten Bereiche, in denen das weichere, verformbare Material vorgesehen ist.

Bei einem Aufsatz 1 nach Figur 1, der sechs zweite Bereiche mit den Halteelementen 25 und deren Fortsätzen 29 aufweist, sind also vorzugsweise sechs im gleichen Umfangsabstand zueinander angeordnete Vertiefungen im Ringbereich des Fortsatzes vorgesehen. Dies bedeutet aber auch, dass ein Aufsatz 1, der auf einen Fortsatz dieser Art aufgesteckt wird, in einer Drehposition angeordnet sein sollte, in der die zweiten Bereiche mit dem weicheren, verformbaren Material unmittelbar einer zugeordneten Vertiefung gegenüberliegen. Das Aufbringen eines Aufsatzes 1 auf einen Fortsatz wird jedoch dadurch vereinfacht, dass insbesondere vorgesehen ist, dass im Ringbereich des Fortsatzes als mindestens eine Vertiefung eine Ringnut vorgesehen ist, deren Basis einen kleineren Außendurchmesser aufweist, als der unmittelbar in Richtung zum freien Ende des Fortsatzes gesehen anschließende Bereich der Außenfläche. Auf diese Weise wird zwischen der Außenfläche und dem Boden der Ringnut eine Schulter ausgebildet, an welcher der Aufsatz 1 nach dem Aufbringen auf den Fortsatz anliegt. Damit wird ein axiales Abziehen des Aufsatzes von dem Fortsatz mit sehr hoher Sicherheit vermieden.

Da außerdem das weiche Material der zweiten Bereiche des Aufsatzes 1 in die als Ringnut ausgebildete Vertiefung flächig angedrückt wird und damit unmittelbar am Fortsatz anliegt, wird auch mit hoher Sicherheit durch die beim Andrücken und Anformen des weicheren Materials aufgebauten Reibungskräften vermieden, dass der Aufsatz 1 bei einer Einleitung eines Drehmoments sich ungewollt gegenüber der Spritze beziehungsweise Karpule verdreht.

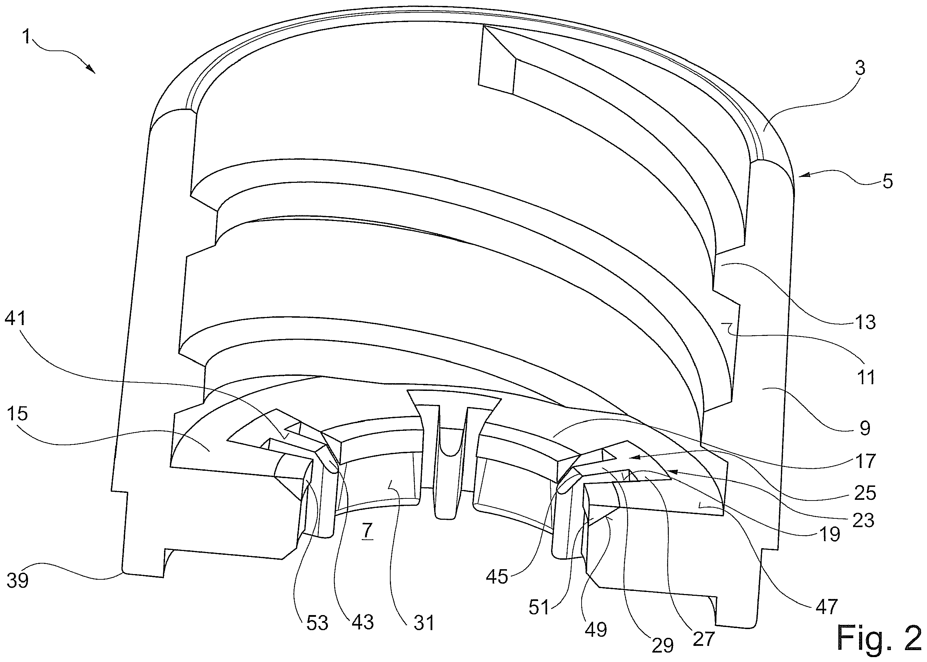

Figur 2 zeigt ein zweites Ausführungsbeispiel eines Aufsatzes 1 in perspektivischer Ansicht, wobei der Aufsatz 1 in Längsrichtung geschnitten ist.

Das in Figur 2 wiedergegebene Ausführungsbeispiel des Aufsatzes entspricht im Wesentlichen dem in Figur 1 dargestellten. Gleiche und funktionsgleiche Teile sind mit identischen Bezugsziffern versehen. Insofern wird auf die Beschreibung zu Figur 1 verwiesen.

Zunächst wird auf die Übereinstimmungen des in Figur 2 wiedergegebenen zweiten Ausführungsbeispiels eingegangen, die sich bezüglich des ersten Ausführungsbeispiels gemäß Figur 1 ergeben:
Der Aufsatz 1 hat eine Stirnseite 3, die in Figur 1 in Draufsicht wiedergegeben wurde. Der Grundkörper 5 des Aufsatzes 1 weist einen Hülsenabschnitt 9 auf, der in der Regel zylindrisch ausgebildet ist und auf seiner Innenfläche 11 mit einem Gewinde 13 versehen ist. An dem der Stirnseite 3 gegenüberliegenden Ende 39 des Grundkörpers ist der Ringkörper 15 vorgesehen, der mindestens ein Segment 17 und wenigstens eine Ausnehmung 19 aufweist, wie es bei der Grundform des anhand von Figur 1 erläuterten Aufsatzes 1 der Fall ist. Vorzugsweise ist aber auch bei dem Ausführungsbeispiel gemäß Figur 2, wie bei dem Aufsatz 1 nach Figur 1 vorgesehen, dass der Ringkörper 15 sechs, in Umfangsrichtung gesehen, in gleichem Abstand zueinander angeordnete Segmente 17 und sechs dazwischen liegende Ausnehmungen 19 aufweist. Auch hier ist eine Haltevorrichtung 23 vorgesehen, die bei der Grundform des Aufsatzes 1 ein Halteelement 25 umfasst, hier bei dem Ausführungsbeispiel nach Figur 2 sechs derartige Halteelemente 25, die jeweils in einer Ausnehmung 19 angeordnet sind.

Figur 2 zeigt, dass das Halteelement 25 außer dem Grundelement 27 auch den Fortsatz 29 aufweist, der mittig, wie bei dem Ausführungsbeispiel gemäß Figur 1, von dem Grundelement 27 entspringt und in radialer Richtung, also in Richtung auf das in Figur 2 nicht wiedergegebene Zentrum 21 des Freiraums 7 verläuft.

Auch bei dem Ausführungsbeispiel gemäß Figur 2 bilden die Segmente 17 erste Bereiche aus einem ersten Material und die Halteelemente 25 zweite Bereiche aus einem zweiten Material, wobei die Materialeigenschaften identisch sind, wie anhand von Figur 1 erläutert.

Das Ausführungsbeispiel des Aufsatzes 1 gemäß Figur 2 unterscheidet sich in folgenden Aspekten von dem Ausführungsbeispiel nach Figur 1:
Die in Richtung auf die Stirnseite 3 weisende Oberfläche 41 der Fortsätze 29 weist in ihrem dem Freiraum 7 zugewandten Endbereich eine Schrägfläche 43 auf, die ausgehend von der Oberfläche 41 über einen Knick 45 nach unten in Richtung auf das Ende 39 des Aufsatzes 1 abfällt.

Es sei hier darauf hingewiesen, dass eine derartige Schrägfläche sehr wohl auch bei dem Ausführungsbeispiel nach Figur 1 vorgesehen werden kann.

Das Ausführungsbeispiel des Aufsatzes 1 gemäß Figur 2 unterscheidet sich auch noch dadurch, dass die der Stirnseite 3 zugewandte Oberseite 47 des mindestens einen Segments 17, hier aller Segmente 17, in einem dem Freiraum 7 zugewandten Bereich eine Schräge 49 aufweist, die von der Oberseite 47 abfällt in einer Richtung, die von der Stirnseite 3 weggerichtet ist. Auf die Schräge 49 ist ein Keil 51 aufgesetzt, der Teil der Haltevorrichtung 23 ist. Er besteht aus dem zweiten Material, welches weicher ist als das formstabile erste Material und außerdem verformbar. Ein derartiger Keil 51 ist auf mindestens einem der Segmente 17 vorgesehen, vorzugsweise jedoch auf allen, sodass sechs ringsegmentartig ausgebildete Keile 51 an den Enden aller Segmente 17 vorhanden sind.

Die der Stirnseite 3 zugewandte Oberseite der Keile 51 stellt eine Fortsetzung der Oberseite 47 der Segmente 17 dar.

Vorzugsweise ist vorgesehen, dass eine dem Freiraum 7 zugewandte Vorderseite 53 des Keils 51 über die jeweilige Innenseite 31 eines zugehörigen Segments 17 in Richtung auf das Zentrum 21 vorspringt.

Diese Ausgestaltung ist vorzugsweise bei allen sechs Segmenten 17 vorgesehen, was allerdings nicht zwingend erforderlich ist.

Für das erste und zweite Ausführungsbeispiel nach den Figuren 1 und 2 gilt, dass vorzugsweise sämtliche Segmente 17, Ausnehmungen 19 und alle Elemente der Haltevorrichtung 23 identisch ausgebildet sind, also die Halteelemente 25, das Grundelement 27, der Fortsatz 29 sowie der Keil 51, der allerdings lediglich in dem Ausführungsbeispiel gemäß Figur 2 vorgesehen ist.

Die Keile 51 ergeben für das weichere Material eine gegenüber dem Ausführungsbeispiel nach Figur 1 größere Anlagefläche an dem Fortsatz. Die als Keilflächen wirkenden Schrägflächen 43 drängen das weichere Material der Keile 51 verstärkt gegen den Fortsatz und verpressen es in der mindestens einen Vertiefung, wenn versucht werden sollte, den Aufsatz 1 von dem Fortsatz abzuziehen. Es ergibt sich also ein verbesserter Halt des Aufsatzes bei axial wirkenden Zugkräften.

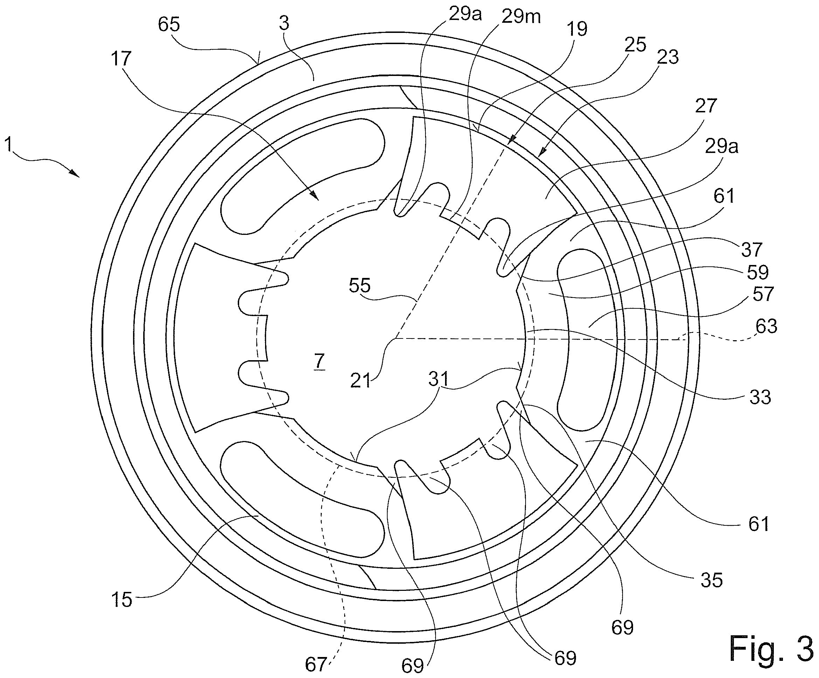

Figur 3 zeigt in Draufsicht ein weiteres Ausführungsbeispiel eines Aufsatzes 1, wobei auch in Figur 3, wie in Figur 1, dem Betrachter die Stirnseite 3 des Aufsatzes 1 zugewandt ist. Gleiche und funktionsgleiche Teile sind mit den Bezugszeichen versehen, wie sie auch zu den vorangegangenen Figuren verwendet wurden. Insofern wird auf die Beschreibung der Figuren 1 und 2 verwiesen.

Auch das Ausführungsbeispiel des Aufsatzes 1 nach Figur 3 kann so ausgebildet sein, dass es lediglich ein Segment 17 und nur eine Aussparung 19 aufweist. Vorzugsweise ist jedoch vorgesehen, dass das Ausführungsbeispiel des Aufsatzes 1 gemäß Figur 3 drei in gleichem Umfangsabstand zueinander angeordnete Segmente 17 und drei dazwischen liegende, auch als Taschen bezeichnete Ausnehmungen 19 aufweist. Auch dieser Aufsatz weist eine Haltevorrichtung 23 auf, die bei der Grundform des Aufsatzes 1 ein in der einen Ausnehmung 19 des Aufsatzes 1 vorgesehenes Halteelement 25 umfasst. Hier sind es entsprechend drei Halteelemente 25, die jeweils in den drei Ausnehmungen 19 untergebracht sind.

Vorzugsweise sind alle Halteelemente 25 identisch ausgebildet. Jedes Halteelement umfasst ein Grundelement 27, welches an dem dem Zentrum 21 abgewandten Boden der Ausnehmung 19 anliegt und vorzugsweise kreissegmentartig aufgebaut ist. In Abweichung zu dem Ausführungsbeispiel des Aufsatzes 1 gemäß den Figuren 1 und 2 ist hier vorgesehen, dass von dem Grundelement 27 zwei symmetrisch zu einer Mittelachse 55 des Halteelements 25 angeordnete Fortsätze 29a vorgesehen sind, die, wie die Fortsätze der Ausführungsbeispiele gemäß den Figuren 1 und 2, ein in den Freiraum 7 ragendes Ende aufweisen, welches insbesondere abgerundet ist, wie dies auch bei den Ausführungsbeispielen gemäß den Figuren 1 und 2 der Fall ist.

Außerdem weist das Grundelement 27 gemäß Figur 3 einen mittleren Vorsprung 29m auf, dessen Stirnseite symmetrisch zu der das Zentrum 21 schneidenden Mittelachse 55 ausgebildet ist und vorzugsweise kreissegmentförmig gebogen ist; es könnte auch eben ausgebildet sein, wie dies oben beschrieben wurde

Nicht nur die Halteelemente 25, sondern auch die Segmente 17 des Aufsatzes 1 gemäß Figur 3 sind alle identisch aufgebaut. Hier ist vorgesehen, dass die Segmente 17 mindestens einen vorzugsweise ringsegmentförmig ausgebildeten Hohlraum 57 aufweisen, der mit Luft, oder vorzugsweise mit dem anhand der Figuren 1 und 2 erläuterten zweiten Material gefüllt ist. Ein Segment kann auch mehrere vorzugsweise nebeneinander liegende Hohlräume aufweisen.

Der Hohlraum 57 ist durch einen kreisbogenförmigen Wandabschnitt 59 gegenüber dem Freiraum 7 abgegrenzt. Außerdem von den - in Umfangsrichtung gesehenen - Enden des Wandabschnitts 59 ausgehenden Wandbereichen 61, die vorzugsweise spiegelbildlich ausgebildet sind zu einer Mittelachse 63 des Segments 17, die das Zentrum 21 schneidet.

Die Innenseite 31 eines jeden Segments 17 ist in einem mittleren Bereich 33 vorzugsweise kreisbogenförmig ausgebildet, und in spiegelbildlich zur Mittelachse 63 in daran links und rechts anschließenden Seitenbereichen 35 nach außen geneigt, das heißt, dass die Seitenbereiche 35 und 37 gegenüber dem mittleren Bereich 33 nach außen abfallen, also in Richtung zur äußeren Umfangsfläche 65 des Aufsatzes 1.

Auch bei dem Ausführungsbeispiel nach Figur 3 ist vorgesehen, dass das mindestens eine Segment 17 beziehungsweise die drei Segmente 17 Teil eines Ringkörpers 15 sind, wobei die Segmente 17 und die Ausnehmungen 19 mit den Halteelementen 25 entlang einer gedachten Umfanglinie mindestens einen ersten durch die Segmente 17 gebildeten Bereich mit einem ersten Material und wenigstens einen durch die Halteelement 25 gebildeten zweiten Bereich mit einem zweiten Material realisieren.

In Figur 3 ist eine gestrichelte Linie 67 eingezeichnet, die den Verlauf der Außenfläche eines Fortsatzes einer Spritze, Karpule oder dergleichen verdeutlicht, und zwar im Bereich der mindestens einen Vertiefung des Fortsatzes, die vorzugsweise als Ringnut ausgebildet ist. Dabei bildet die Linie 67 den Boden dieser Ringnut ab.

Es zeigt sich, dass der diametral gemessene Abstand einer Innenseite 31 eines Segments 17 zu einer dem Zentrum 21 zugewandten Innenfläche des Fortsatzes 29m kleiner ist als der Innendurchmesser der Linie 67. Entsprechend verliefe auch die Außenfläche des Fortsatzes bei dem Ausführungsbeispiel des Aufsatzes 1 gemäß Figur 1.

Es zeigt sich, dass beim Aufstecken des Aufsatzes 1 auf einen Fortsatz die Innenseiten 31 der Segmente 17 und die dem Zentrum 21 zugewandten Enden des Fortsatzes 29 beziehungsweise der Fortsätze 29a und 29m gegenüber dem Zentrum 21 radial nach außen gedrängt werden. Dabei ergibt sich Folgendes:

Bei einer von dem Zentrum 21 radial nach außen auf die Wandabschnitte 59 wirkenden Kraft werden die seitlichen Wandbereiche 61 der Segmente 17 verformt, nämlich von der Mittelachse 63 nach außen in Richtung auf die seitlich angrenzenden Ausnehmungen 19. Diese Auswärtsbewegung der Wandbereiche 61 ist besonders ausgeprägt, wenn der Hohlraum 57 mit dem zweiten verformbaren Material ausgefüllt ist, aus dem auch die Halteelemente 25 bestehen.

Durch die nach außen gedrängten Wandbereiche 61 wird also der Freiraum der Ausnehmungen 19 seitlich eingeengt, sodass die in den Ausnehmungen 19 untergebrachten Halteelemente 25 komprimiert werden. Das zweite Material, welches die Halteelemente 25 aufweisen beziehungsweise aus denen die Halteelemente 25 vorzugsweise bestehen, wird dadurch nach innen in Richtung Zentrum 21 verdrängt und in die mindestens eine Vertiefung in der Außenfläche des Fortsatzes eingeformt. Um zu vermeiden, dass in Umfangsrichtung verteilt jeweils einem Halteelement 25 eine Vertiefung zugeordnet werden muss, was eine korrekte Lageausrichtung des Aufsatzes 1 gegenüber dem Fortsatz bedingt, ist die mindestens eine Vertiefung vorzugsweise als umlaufende Ringnut in der Außenfläche des Fortsatzes ausgebildet, wie dies bereits oben festgehalten wurde.

Bei dem Aufstecken des Aufsatzes 1 auf einen Fortsatz werden auch radial nach außen, also von dem Zentrum 21 weg gerichtete Kräfte auf das Halteelement 25 ausgeübt, sodass dessen mindestens einer Vorsprung, hier also die Vorsprünge 29a und der Vorsprung 29m nach außen gedrängt werden. Dies führt dazu, dass Freiräume 69 seitlich neben den Fortsätzen 29a und in der dem Zentrum 21 zugewandten Innenseite der Halteelemente 25 vorgesehene Aussparungen 69 mit dem von innen nach außen und außen nach innen verdrängten zweiten Material des Halteelements 25 aufgefüllt werden, wobei das verdrängte Material der Halteelemente 25 auch in die mindestens eine Vertiefung beziehungsweise Ringnut in der Außenfläche des Fortsatzes eingeformt beziehungsweise eingepresst wird.

In den von den Halteelementen 25 realisierten zweiten Bereichen des Aufsatzes 1 wird also ein verformbares zweites Material gegen die Außenfläche des Fortsatzes gedrängt, nämlich in den Bereich der vorzugsweise ringförmigen Vertiefung. Dieses relativ weiche Material baut relativ hohe Reibungskräfte mit dem Fortsatz auf, sodass zum einen eine sehr gute Sicherung gegen ein axiales Abziehen des Aufsatzes 1, aber auch gegen eine Relativdrehung dieses Aufsatzes 1 gegenüber dem Fortsatz gewährleistet ist. Dies insbesondere deshalb, weil bei Ausgestaltung einer Ringnut in der Außenfläche des Fortsatzes eine in Richtung der Außenfläche vorspringende Schulter ausgebildet wird, die ein Abziehen des Aufsatzes in Richtung zum freien Ende des Fortsatzes sicher verhindert.

Darüber hinaus zeigt sich, dass die Seitenbereiche 35 und 37 der Segmente 17 jeweils eine Keilfläche bilden, durch die bei Einleitung eines Drehmoments in den Aufsatz 1 das zweite Material verstärkt gegen die Außenfläche des Fortsatzes beziehungsweise in die mindestens eine Vertiefung hineingedrängt und in dieser verpresst wird. Die Reibungskräfte zwischen Aufsatz 1 und dem Fortsatz einer Spritze oder Karpule werden also durch die geneigten Seitenbereiche 35 und 37 noch verstärkt.

Derselbe besonders bevorzugte Effekt stellt sich auch bei dem Ausführungsbeispiel nach Figur 1 ein, wo die Innenseite 31 des mindestens einen Segments beziehungsweise der Segmente 17 ebenfalls zwei Seitenbereiche 35 und 37 aufweist, die keilförmig gegenüber dem mittleren Bereich 33 verlaufen und in die das Material des Fortsatzes 29 des Halteelements 25 beim Aufstecken eines Aufsatzes 1 auf einen Fortsatz gedrängt wird. Auch hier wird also bei Einleitung eines Drehmoments in den Aufsatz 1 nach Figur 1 eine erhöhte Reibkraft aufgebaut, die den Aufsatz gegen eine Relativdrehung gegenüber dem Fortsatz sichert.

Oben wurde ausgeführt, dass die Segmente 17 und die Ausnehmungen 19 sich in Richtung auf den Freiraum 7 verjüngen. Dies ist allerdings nicht zwingend erforderlich. Es können sehr wohl auch andere bevorzugte Ausgestaltungen gewählt werden, bei denen sich Segmente 17 oder Ausnehmungen 19 in Richtung auf den Freiraum erweitern. Figur 3 zeigt eine Ausführungsvariante, bei der sich die Ausnehmungen 19 ausgehend von ihrer äußeren Grundfläche zunächst in Richtung auf den Freiraum 7 verjüngen, dann aber breiter werden. Entsprechend sind die Außenflächen der in den Ausnehmungen 19 liegenden Grundelemente 27 ausgebildet. Komplementär dazu sind die Außenflächen der angrenzenden Segmente 17 ausgebildet.

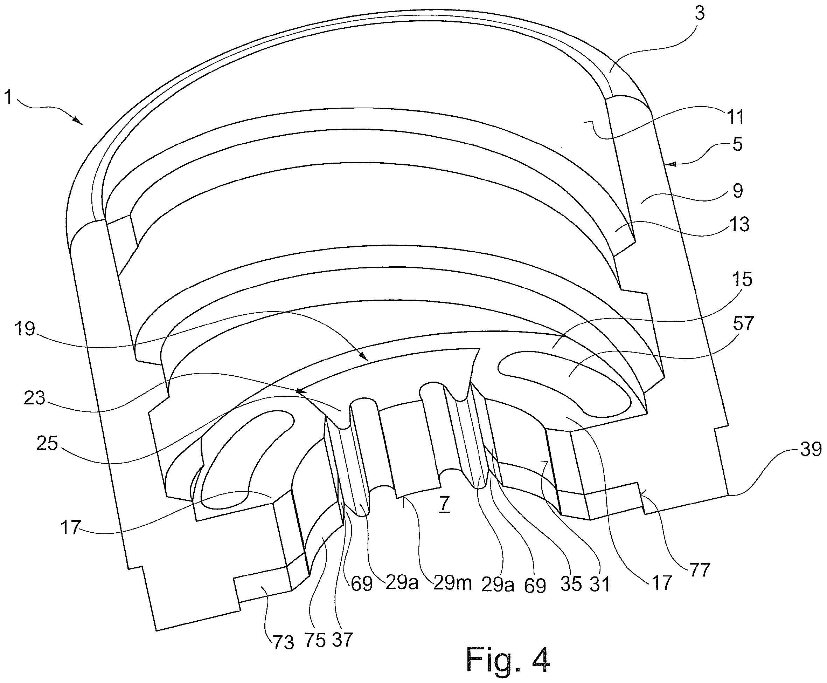

Figur 4 zeigt den Aufsatz 1 gemäß Figur 3 in perspektivischer Ansicht, wobei der Aufsatz 1 in Längsrichtung aufgeschnitten ist. Gleiche und funktionsgleiche Teile sind mit den Bezugszeichen versehen, wie sie auch bei den vorangegangenen Figuren verwendet wurden.

Figur 4 zeigt, dass der Aufsatz 1 eine Stirnseite 3 sowie einen Grundkörper 5 aufweist, von dessen Innenfläche 11 ein Gewinde 13 ausgeht. Dieses dient dazu, in den Aufsatz 1 den Ansatz einer Kanüle einzuschrauben, die dadurch fest mit einer Spritze, Karpule oder dergleichen verbunden wird. Beim Aufschrauben der Kanüle soll verhindert werden, dass sich der Aufsatz 1 gegenüber dem Fortsatz verdreht und damit eine sichere Befestigung der Kanüle gewährleistet wird.

Die Schnittdarstellung lässt erkennen, dass der Aufsatz 1 einen Hülsenabschnitt 9 sowie einen Ringabschnitt 15 aufweist, der am unteren Ende 39 des Grundkörpers 5 des Aufsatzes 1 angeordnet ist. Deutlich erkennbar sind hier zwei Segmente 17 und eine dazwischen liegende Ausnehmung 19, in die das Halteelement 25 der Haltevorrichtung 23 eingebracht sind.

Die perspektivische Ansicht zeigt deutlich keilförmige Freiräume 69, die rechts und links neben den seitlichen Fortsätzen 29a des Halteelements 25 vorhanden sind, und die von den Seitenbereichen 35 und 37 der Innenseite 31 des Segments 17 begrenzt werden.

Figur 4 lässt auch die Hohlräume 57 in den Segmenten 17 erkennen.

Die perspektivische Darstellung dieser Figur zeigt darüber hinaus, dass die Haltevorrichtung 23 vorzugsweise noch ergänzt werden kann durch eine von unten an den Aufsatz 1 im Bereich des Endes 39 angesetzte Schicht 73, die verformbares zweites Material umfasst, vorzugsweise aus diesem besteht. Vorzugsweise kann im Übrigen noch vorgesehen werden, dass die Vorderseite 75 dieser Schicht gegenüber der Innenseite 31 der Segmente 17 vorspringt in Richtung auf das hier nicht dargestellte Zentrum 21. Die Schicht 73 wird vorzugsweise auf der dem Zentrum 21 beziehungsweise dem Freiraum 7 abgewandten Seite durch eine Stufe 77 des Ringkörpers 15 begrenzt, welche die Schicht 73 stützt, sodass deren zweites Material beim Aufsetzen des Aufsatzes 1 auf einen Fortsatz gegen die mindestens eine Vertiefung in der Außenfläche des Fortsatzes, vorzugsweise in die Ringnut des Fortsatzes gedrängt wird. Dadurch baut die Haltevorrichtung 23 noch zusätzliche Reibungskräfte auf, die den Aufsatz 1 gegen Verdrehen gegenüber dem Fortsatz sichern.

Mit Hinweis auf das Ausführungsbeispiel gemäß Figur 2 gilt Folgendes:
Zum einen ist es möglich, den in Figur 2 dargestellten Keil 51 der Haltevorrichtung 23 auch bei dem Aufsatz 1 gemäß Figur 4 vorzusehen, entweder statt der Schicht 73 oder zusätzlich dazu.

Entsprechend kann bei dem Ausführungsbeispiel gemäß Figur 2 der Keil 51 ergänzt werden um eine Schicht 73 oder durch diese ersetzt werden.

Schließlich ist es auch noch möglich, bei dem in Figur 4 dargestellten Ausführungsbeispiel bei dem Halteelement 25 mindestens eine Schrägfläche vorzusehen, die in Figur 2 mit der Bezugsziffer 43 gekennzeichnet wurde.

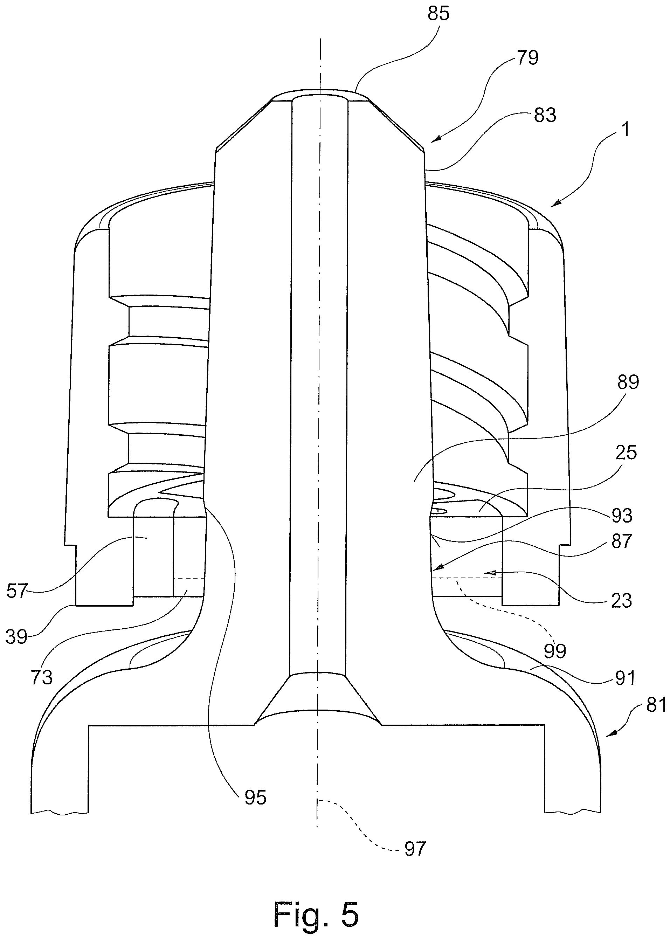

Figur 5 zeigt im Längsschnitt einen Aufsatz 1, der auf einen Fortsatz 79 einer Spritze, einer Karpule 81 oder dergleichen aufgebracht ist. Beim Aufbringen eines Aufsatzes nach den vorangegangenen Figuren 1 bis 4 ergeben sich identische Verhältnisse, sodass die Erläuterungen zu dem Aufsatz nach Figur 5 in Zusammenhang mit dem Fortsatz 79 für alle Ausführungsbeispiele gelten. Dabei kann, wie oben ausgeführt, der Fortsatz auch Teil eines Pens oder eines Adapters sein, der als Anschluss für ein medizinisches Gerät oder einen Schlauch ausgebildet ist.

Bezüglich der Details zu dem Aufsatz 1 wird verwiesen auf die Erläuterungen zu den Figuren 3 und 4.

Der Fortsatz 79 weist eine Außenfläche 83 auf, in die in einem Abstand zum freien Ende 85 des Fortsatzes 79 mindestens eine Vertiefung 87 eingebracht ist. Vorzugsweise ist vorgesehen, dass diese Vertiefung als Ringnut ausgebildet ist, deren Außendurchmesser kleiner ist als der Außendurchmesser der Außenfläche in einem oberhalb der Ringnut liegenden Bereich 89 des Fortsatzes 79. Mit "oberhalb" wird hier Bezug genommen auf die Darstellung gemäß Figur 5, in der das distale Ende 91 einer Spritze oder Karpule 81 gemäß der Ansicht in Figur 5 oben angeordnet ist, sodass der Fortsatz 79 von hier nach oben verläuft.

Dadurch, dass im Bereich der Vertiefung 87 ein kleinerer Außendurchmesser gegeben ist, wird gegenüber dem Boden 93 der Vertiefung 87 eine nach außen vorspringende Schulter 95 gebildet, die ein Abziehen des Aufsatzes 1 nach oben in Richtung der Mittelachse 97 des Fortsatzes 79 verhindert.

In Zusammenhang mit Figur 4 wurde ausgeführt, dass im Bereich des Endes 69 eine Schicht 73 vorgesehen ist, welche Teil der Haltevorrichtung 23 ist. Aus der hier gewählten Schnittdarstellung des Aufsatzes 1 wird deutlich, dass die Schicht 73, die hier durch eine gestrichelte Linie 99 abgegrenzt ist, auch integraler Bestandteil des Halteelements 25 und/oder des Hohlraums 57 sein kann.

Aus Figur 5 ist ersichtlich, dass der Ringbereich 15 eines Aufsatzes 1 nach dem Aufstecken auf einen Fortsatz 79 im Bereich einer Ringfläche des Fortsatzes angeordnet ist, die oberhalb des distalen Endes 91 der Spritze oder Karpule liegt. Innerhalb dieser Ringfläche des Fortsatzes 79 ist die mindestens eine Vertiefung 87 vorgesehen, vorzugsweise die oben angesprochene Ringnut. Im Bereich der Ringfläche des Fortsatzes liegen nach dem Aufsetzen des Aufsatzes 1 auf den Fortsatz 97 die ersten und zweiten Bereiche des Aufsatzes, die zum einen durch die Segmente 17 und zum anderen durch die Halteelemente 25 realisiert werden. Hier im Bereich der Ringfläche des Fortsatzes 79 wird das zweite Material des Aufsatzes 1, welches verformbar ist, an den Fortsatz 79 angeformt, sodass dieses unmittelbar an dem Fortsatz 79 anliegt und diesen durch hohe Reibkräfte gegen axiales Abziehen und gegen eine Verdrehung gegenüber dem Fortsatz 79 sichert.

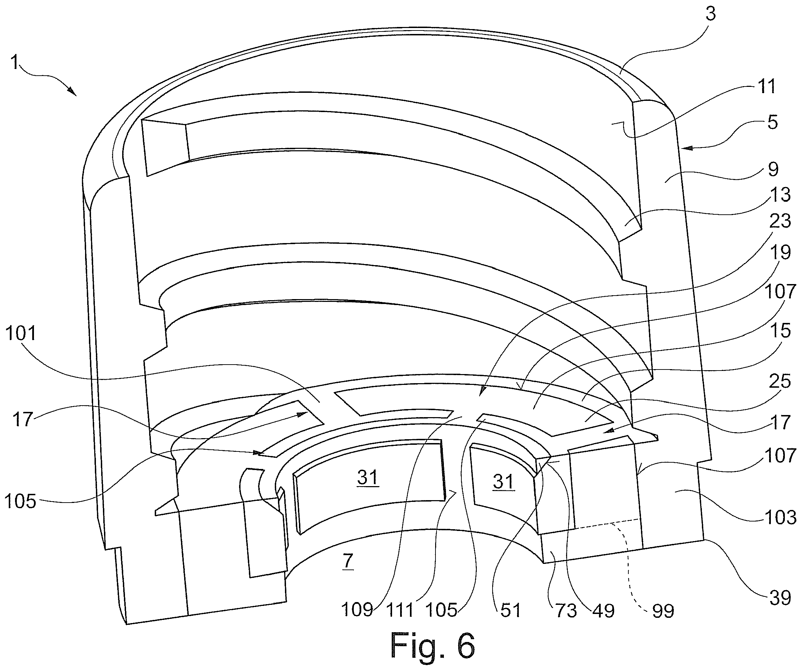

Figur 6 zeigt in perspektivischer Ansicht einen Längsschnitt durch ein weiteres Ausführungsbeispiel eines Aufsatzes 1. Gleiche und funktionsgleiche Teile sind mit identischen Bezugsziffern versehen, sodass auf die vorangegangene Beschreibung verwiesen wird.

Der Aufsatz 1 weist einen Grundkörper 5 mit einer Stirnseite 3 und einen Hülsenabschnitt 9 mit einer Innenfläche 11 auf, auf der ein Gewinde 13 vorgesehen ist. Der Aufsatz 1 hat außerdem einen Ringkörper 15, der mindestens ein Segment 17 und mindestens eine Ausnehmung 19 umfasst. Auch hier ist vorgesehen, dass der Aufsatz 1 in gleichmäßigen Abständen zueinander angeordneten Segmente 17 und dazwischen liegende Ausnehmungen 19 aufweist.

Auch der hier dargestellte Aufsatz 1 weist eine Haltevorrichtung 23 mit einem in einer Ausnehmung 19 untergebrachten Halteelement 25 auf, wobei vorzugsweise jeder Ausnehmung 19 ein Halteelement 25 zugeordnet ist.

Die Segmente 17 weisen eine Innenseite 31 auf, die dem Innenraum 7 zugewandt ist und in Richtung auf das hier nicht dargestellte Zentrum 21 weist. Jedes Segment 17 umfasst einen Steg 101, über den es mit einer Außenwand 103 des Ringsegments 15 verbunden ist. Der Steg 101 verläuft radial in Richtung auf das Zentrum 21 und weist ein Wandsegment 105 auf, welches seitlich gegenüber dem Steg 101 vorsteht, vorzugsweise symmetrisch rechts und links davon.

Die Wandsegmente 105 zweier benachbarter Stege 17 verlaufen entlang einer gedachten Umfangslinie, enden jedoch in einem Abstand zueinander, sodass ein Hohlraum 107 ausgebildet ist, der vorzugsweise mit dem oben erwähnten zweiten Material gefüllt ist, während zumindest das Segment 17, vorzugsweise der gesamte übrige Aufsatz 1 das erste Material umfasst, vorzugsweise aus diesem besteht.

Die Breite der Stege 101 der Segmente 17 ist wesentlich kleiner als die in Umfangsrichtung gemessene Breite der Wandsegmente 105. Beispielsweise ist die Breite der Wandsegmente 105 zwei- bis zehnmal größer als die Breite der Stege 101, vorzugsweise fünf- bis siebenmal. Die Stege 101 und die Wandsegmente 105 sind also im weitesten Sinne T-förmig ausgebildet.

Die Hohlräume 107 weisen das zweite Material auf; sie sind vorzugsweise vollständig damit ausgefüllt, wobei das zweite Material durch zwischen den Enden der Wandsegmente 105 liegende Zwischenräume 109 sich bis zum Freiraum 7 erstreckt.

Unterhalb der Segmente 17 kann vorzugsweise eine Schicht 73 vorgesehen werden, die das zweite Material umfasst, vorzugsweise aus diesem besteht. Durch eine gestrichelte Linie 99 ist angedeutet, dass die Schicht 73 integraler Bestandteil des zweiten Materials im Hohlraum 107 sein kann.

Statt der Schicht 73, vorzugsweise zusätzlich ist auch hier ein Keil 51 aus dem zweiten Material vorgesehen, welcher an einer Schräge 49 der Segmente 17 vorgesehen ist. Auch der Keil 51 weist das zweite Material auf und besteht vorzugsweise aus diesem. Der Keil 51 und die Schicht 73 sind Teil der Haltevorrichtung 23.

Figur 6 lässt erkennen, dass die Innenseite 31 der Segmente 17 gegenüber der dem Innenraum 7 zugewandten Innenfläche 111 des zweiten Materials vorzugsweise etwas vorspringt. Diese Ausgestaltung hat den Vorteil, dass beim Aufbringen des Aufsatzes 1 auf einen Fortsatz 79 der durch die Innenflächen 31 der Segmente 17 gebildete erste Bereich des Aufsatzes 1 mit der Außenfläche des Fortsatzes 79 in Eingriff tritt, sodass das zweite Material, welches weicher ist als das erste Material im ersten Bereich beim ersten Aufschieben des Aufsatzes 1 nicht mit dessen Außenfläche in Berührung kommt und durch die Reibungskräfte abgetragen wird.

Der Aufsatz 1 nach Figur 6 weist also die genannten ersten Bereiche mit dem ersten Material auf und außerdem zweite Bereiche, die durch das zweite Material gebildet werden.

Im aufgesetzten Zustand des Aufsatzes 1 wirken vom Zentrum 21 aus radial nach außen gerichtete Kräfte, sodass die Wandsegmente 105 nach außen in Richtung auf die Außenwand 103 des Aufsatzes 1 gedrängt werden. Dadurch wird das in den Hohlräumen 107 vorhandene zweite Material komprimiert und aus den Zwischenräumen 109 und im Bereich der Schicht 73 nach innen in den Innenraum 7 gedrängt. Diese Verlagerung des zweiten Materials führt dazu, dass dieses in aufgesetztem Zustand gegen die mindestens eine Vertiefung, hier also gegen die ringförmige Vertiefung 87 in der Außenfläche 93 des Fortsatzes 79 (siehe Figur 5) gedrängt und in die Vertiefung eingeformt, das heißt in dieser verpresst wird.

Dadurch ergeben sich hohe Reibungskräfte, aufgrund derer der Aufsatz 1 nicht nur in axialer Richtung am Vorsprung 79 gesichert ist, wobei die Schulter 95 besonders guten axialen Halt verleiht, sondern auch ein Schutz gegen ein ungewolltes Verdrehen des Aufsatzes 1 gegenüber dem Fortsatz 79 gegeben ist.

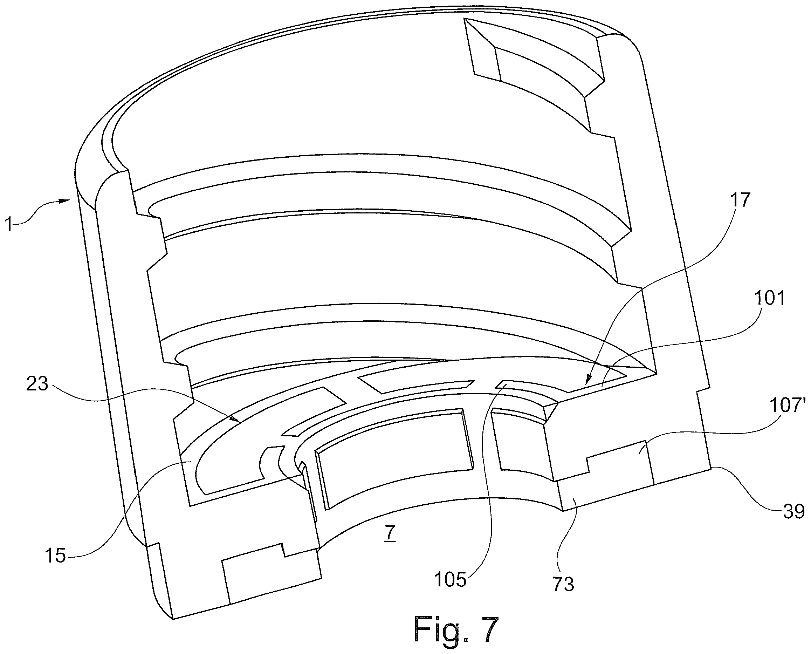

Figur 7 zeigt schließlich den Aufsatz aus Figur 6 in perspektivischer Ansicht im Längsschnitt, wobei allerdings die Schnittebene in einer anderen Ebene liegt, als dies in Figur 6 der Fall ist.

Gleiche und funktionsgleiche Teile sind mit gleichen Bezugsziffern versehen, sodass insofern auf die vorangegangene Beschreibung verwiesen wird.

Aus der Schnittebene gemäß Figur 7 ist ersichtlich, dass die Stege 101 der Segmente 17 sich vorzugsweise nicht über die komplette Höhe des Ringkörpers 15 erstrecken, sondern vielmehr in einem Abstand zum Rand 39. Dabei kann vorzugsweise vorgesehen werden, dass der Abstand des Stegs 101 zum unteren Rand etwas größer ist als der Abstand des Wandsegments 105. Wird das Wandsegment 105 beim Aufbringen des Aufsatzes 1 auf einen Fortsatz 79 einer Kanüle oder Spritze 81 in radialer Richtung nach außen verlagert, so wird auch hier im Bereich der Schicht 73 der Haltevorrichtung 23 ein Hohlraum 107' gebildet, in welchem das Material der Schicht 73 komprimiert und damit nach vorne in Richtung auf den Freiraum 7 gedrängt wird. Dies verbessert die Anlage des zweiten Materials, welches die Haltevorrichtung 23 aufweist beziehungsweise aus welchem die Haltevorrichtung 23 besteht. Diese Ausgestaltung verbessert also das Anformen des zweiten Materials der Schicht 23 an die mindestens eine Vertiefung beziehungsweise an die vorzugsweise vorgesehene Ringnut 87 des Vorsprungs 79.

Aus den Erläuterungen zu den Figuren wird Folgendes deutlich:

Der Aufsatz 1 in seinen verschiedenen Ausführungsformen weist einen Grundkörper auf, der zwei Materialien umfasst, vorzugsweise aus diesen besteht. Ein erstes Material ist formstabil und ein zweites weicher als dieses Material, außerdem verformbar. Der Grundkörper 5 des Aufsatzes 1 weist einen Ringbereich 15 mit einem ersten Bereich und einem zweiten Bereich auf, wobei der erste Bereich durch Segmente und der zweite Bereich durch Halteelemente 25 einer Haltevorrichtung gebildet werden, und wobei die Segmente aus dem ersten Material und die Halteelemente 25 aus dem zweiten Material bestehen. Der Ringbereich 15 bildet damit einen Eingriffsbereich, der im aufgesetzten Zustand des Aufsatzes 1 auf einem Fortsatz 79 einer Spritze, einer Karpule oder dergleichen in mindestens eine Vertiefung 87 in der Außenfläche 83 des Fortsatzes 79 eingreift. Der Aufsatz zeichnet sich dadurch aus, dass das zweite Material in die mindestens eine Vertiefung eingeformt beziehungsweise eingepresst wird, oder, in anderen Worten, mit dieser verpresst wird. Dadurch werden hohe Reibungskräfte erzeugt, die den Aufsatz sicher am Fortsatz halten.

Besonders bevorzugt wird der Aufsatz 1 in einem Zwei-Komponenten-Spritzgussverfahren hergestellt, wobei die ersten und zweiten Materialien hierbei eingesetzt werden. Die Figuren zeigen, dass die ersten und zweiten Bereiche verschiedene Konturen aufweisen, und dass die Haltevorrichtung 23 verschiedene Teilelemente umfasst, nämlich das Halteelement 25, den Keil 51 und/oder die Schicht 73. Der Grundkörper 5 des Aufsatzes 1 weist vorzugsweise das erste Material auf, insbesondere besteht er aus diesem. Entsprechend weist das Halteelement 25 das zweite Material auf und besteht vorzugsweise aus diesem. Es ist also möglich, die verschiedenen Konturen des Aufsatzes 1, wie sie aus den Figuren 1 bis 7 ersichtlich sind, in einem einfachen Zwei-Komponenten-Spritzgussverfahren herzustellen, wobei die beiden Materialien ineinander geformt werden. Damit ist die Realisierung des Aufsatzes 1 relativ kostengünstig.

Vorzugsweise umfasst das erste Material temperaturbeständige, insbesondere hochtemperaturbeständige Kunststoffe, vorzugsweise Polycarbonate, Polysulfone oder Polypropylene, oder besteht nur aus diesen Kunststoffen. Vorzugsweise besteht das zweite Material aus Weichkunststoffen, insbesondere thermoplastischen Elastomeren, wie TPE (Thermoplastisches Elastomer), PTFE (Polytetrafluorethylen), Weich-PVC (Polyvinylchlorid) oder Silikone. Insbesondere besteht das zweite Material vollständig aus diesen Substanzen. Für das erste und zweite Material können auch Hart- beziehungsweise Weich-Copolymere verwendet werden.

Eine Spritze oder Karpule 81, die mit einem Aufsatz 1 der hier angesprochenen Art verschlossen werden soll, weist einen Innenraum zur Aufnahme einer Substanz auf, die entweder einem Patienten verabreicht werden soll oder aber gewonnen wird, insbesondere von dem Patienten, um diese einer Auswertung oder Behandlung zu unterziehen, um sie gegebenenfalls dem Patienten wieder zu verabreichen.

Der als Spritzenzylinder beziehungsweise Karpulenzylinder bezeichnete Körper weist ein distales Ende 91 auf, von dem ein endständiger Fortsatz 79 entspringt, der eine Außenfläche 83 aufweist. In diese ist mindestens eine Vertiefung 87 eingebracht, und zwar in einem derartigen Abstand zum freien Ende 85 des Fortsatzes 79, dass sie beim Aufsetzen eines Aufsatzes 1 im Bereich einer Ringfläche angeordnet ist, in welchem der Ringkörper 15 des Aufsatzes 1 zu liegen kommt. Vorzugsweise weist der Fortsatz 79 eine durchgehende Ringnut als Vertiefung 87 auf, die in Richtung zu dessen freiem Ende 85 durch eine Schulter 95 begrenzt wird, sodass ein axiales Abziehen in Richtung der Mittelachse 97 des Fortsatzes 79 sehr erschwert wird.

Das zweite Material des Aufsatzes 1 ist derart verformbar, dass es nach dem Aufsetzen in die mindestens eine Vertiefung gedrängt und dort eingeformt wird. Das verformbare zweite Material ist vorzugsweise derart gewählt, dass sich hohe Reibungskräfte zwischen der Außenfläche des Fortsatzes 79 ergeben. Dadurch wird auch ein ungewolltes Verdrehen des Aufsatzes 1 gegenüber dem Fortsatz 79 verhindert.

Vorzugsweise sind Freiräume 69 vorgesehen, die insbesondere durch schräge Seitenbereiche 35 und 37 der Segmente 17 gebildet werden. Hier wird das zweite Material besonders gut gegen die Außenfläche des Fortsatzes 79 angepresst, wobei durch die Keilform sichergestellt ist, dass besonders hohe Kräfte einer versehentlichen Verdrehung des Aufsatzes 1 gegenüber dem Fortsatz 79 wirken.

Der Aufsatz 1 ist vorzugsweise so ausgebildet und dimensioniert, dass er auf herkömmliche Fortsätze 79 aller Art aufgesetzt werden kann, die insbesondere eine konische Außenfläche 83 aufweisen, die sich in Richtung auf das freie Ende 85 des Fortsatzes 79 verjüngt. Daher ist es möglich, die sich aus dem Aufsatz 1 ergebenden Vorteile auch bei herkömmlichen Spritzen und/oder Karpulen zu nutzen.

## Patentansprüche

1. Aufsatz für eine Spritze, eine Karpule (81) oder dergleichen, die
- ein distales Ende (91) und an diesem
- einen endständigen Fortsatz (79) aufweist, wobei
- der Fortsatz (79) eine Außenfläche (83), ein freies Ende (85) und mindestens eine in einem Abstand zu diesem in die Außenfläche eingebrachte Vertiefung (87) aufweist, wobei
- der Aufsatz (1) einen ringförmigen Grundkörper (5) aufweist und auf den Fortsatz (79) aufsetzbar ist, wobei
- der Grundkörper (5) einen Freiraum (7) umschließt, in den der Fortsatz (79) einführbar ist, wobei
- der Grundkörper (5) zwei Materialien umfasst, von denen ein ersten Material formstabil und ein zweites Material weicher als das formstabile erste Material und verformbar ist, wobei
- der Grundkörper (5) mindestens einen Eingriffsbereich aufweist, der in auf dem Fortsatz (79) aufgestecktem Zustand in die mindestens eine Vertiefung (87) eingreift, wobei
- der Grundkörper (5) entlang einer gedachten Umfangslinie erste Bereiche mit dem ersten Material und zweite Bereiche mit dem zweiten Material aufweist, wobei
- das zweite Material in auf den Fortsatz (79) aufgesetztem Zustand in die mindestens eine Vertiefung (87) eingeformt wird, und wobei
- das zweite Material in das erste Material eingebettet ist,
**dadurch gekennzeichnet, dass**
- der Grundkörper (5) an einem Ende (39) einen Ringkörper (15) aufweist, der in auf den Fortsatz (79) aufgebrachtem Zustand einen ringförmigen Bereich des Fortsatzes (79) umgreift, wobei in diesem Bereich des Fortsatzes (79) die mindestens eine Vertiefung (87) angeordnet ist, und dass
- der Ringkörper (15) in Umfangsrichtung entlang der gedachten Umfangslinie, vorzugsweise in gleichem Abstand zueinander angeordnete, die ersten Bereiche bildende Segmente (17) und in den Zwischenräumen zwischen den Segmenten Ausnehmungen (19) aufweist, die das zweite Material aufnehmen und die zweiten Bereiche bilden, wobei
- sich die gedachte Umfangslinie im auf den Fortsatz (79) aufgebrachten Zustand im Bereich der mindestens einen Vertiefung (87) des Fortsatzes (79) befindet.

2. Aufsatz nach Anspruch 1, **dadurch gekennzeichnet, dass** der Grundkörper (5) unter Verwendung des ersten Materials und des zweiten Materials in einem Zwei-Komponenten-Spritzgussverfahren herstellbar ist.

3. Aufsatz nach Anspruch 1 oder 2, **dadurch gekennzeichnet, dass** der Grundkörper (5) das erste Material aufweist und in einem Umfangsbereich mindestens eine Ausnehmung (19) umfasst, in der das zweite Material vorgesehen ist, welches den zweiten Bereich bildet.

4. Aufsatz nach einem der vorhergehenden Ansprüche, **dadurch gekennzeichnet, dass** die mindestens eine Vertiefung (87) ringförmig ausgebildet ist.

5. Aufsatz nach Anspruch 1, **dadurch gekennzeichnet, dass** die Segmente (17) und die Ausnehmungen (19) sich in Richtung auf den Freiraum (7) verjüngen.

6. Aufsatz nach Anspruch 1 oder 5, **dadurch gekennzeichnet, dass** das mindestens eine Segment (17) wenigstens einen vorzugsweise ringförmig ausgebildeten Hohlraum (57) aufweist, der vorzugsweise das zweite Material enthält, wobei das mindestens eine Segment (17) einen kreisbogenförmigen Wandabschnitt (59) umfasst, der den wenigstens einen Hohlraum (57) gegenüber dem Freiraum (7) abgrenzt, und an den Enden des Wandabschnitts (59) von diesem ausgehende Wandbereiche (61), die den wenigstens einen Hohlraum (57) seitlich begrenzen, wobei der Wandabschnitt (59) und die Wandbereiche (61) nachgiebig ausgebildet sind, sodass bei einer vom Freiraum (7) her auf den Wandabschnitt (59) wirkenden Kraft die Wandbereiche (61) seitlich nach außen gedrängt werden und somit die in Umfangsrichtung gemessene Breite der seitlich angrenzenden Ausnehmungen (19) reduzieren.

7. Aufsatz nach Anspruch 1, **dadurch gekennzeichnet, dass** das mindestens eine Segment (17) eine dem Freiraum (7) zugewandte Innenfläche (31) aufweist, die vorzugsweise zwei an einem mittleren Bereich (33) angrenzende Seitenbereiche (35, 37) umfasst.

8. Aufsatz nach einem der vorhergehenden Ansprüche 1 und 5 bis 7, **dadurch gekennzeichnet, dass** das mindestens eine Segment (17) eine Oberseite (47) aufweist, und dass in einem dem Freiraum zugewandten Bereich der Oberseite (47) eine Schräge (49) vorgesehen ist, auf welcher ein Keil (51) aufgesetzt ist.

9. Aufsatz nach Anspruch 1, **dadurch gekennzeichnet, dass** das erste Material Polycarbonate, Polysulfone oder Polypropylene umfasst oder nur aus diesen Kunststoffen besteht und/oder dass das zweite Material Weichkunststoffe, insbesondere thermoplastische Elastomere wie TPE, PTFE, Weich-PVC oder Silikone umfasst oder vollständig aus diesen Materialien besteht.

10. Spritze, Karpule oder dergleichen mit einem distalen Ende (91) und einem an diesem vorgesehenen endständigen Fortsatz (75), der eine Außenfläche (79), ein freies Ende (85) und mindestens eine in einem Abstand zu diesem in die Außenfläche eingebrachte Vertiefung (87) aufweist, **gekennzeichnet durch** einen Aufsatz (1) nach einem der Ansprüche 1 bis 9.

## Claims

1. An attachment for a syringe, a carpule (81) or the like, comprising
- a distal end (91) and
- a terminal extension (79) at the distal end, wherein
- the extension (79) includes an outer surface (83), a free end (85), and at least one depression (87) introduced into the outer surface at a distance to the free end, wherein
- the attachment (1) comprises a base body (5) being placeable onto the terminal extension (79), wherein
- the base body (5) enclosing a free space (7), into which the terminal extension (79) is introduced, wherein
- the base body (5) comprising two materials, being composed of a first material that is dimensionally stable and a second material that is softer than the dimensionally stable first material and that is deformable, wherein
- the base body (5) includes at least one engagement region, which in a state in which the annular base body is plugged onto the terminal extension (79) engages into the at least one depression (87), wherein
- along an imaginary circumferential line, the base body (5) has first regions including the first material, and second regions including the second material, wherein
- in a state in which it is plugged onto the terminal extension (79), the second material is molded into the at least one depression (87), and
- the second material is embedded into the first material,
**characterised in that**
- on a first end (39), the base body (5) includes an annular body (15), which in a state in which it is applied to the terminal extension (79) embraces an annular region of the terminal extension (79), wherein the at least one depression (87) is disposed in the annular region of the terminal extension (79), and that
- wherein the annular body (15) includes in a circumferential direction along the imaginary circumferential, preferably arranged at equal distance to each other, line segments (17) which form the first regions, and laterally adjacent openings (19) in intermediate spaces between the segments, in which the second material is arranged, and which form the second regions, wherein
- in the state in which the annular body is applied to the terminal extension (79), the imaginary circumferential line is located in the region of the at least one depression (87) of the terminal extension (79).

2. The attachment according to claim 1, **characterised in that** the base body (5) is manufactured using a two-component injection molding method by using the first material and the second material.

3. The attachment according to claim 1 or 2, **characterised in that** the base body (5) is composed of the first material, and a circumferential region comprises at least one opening (19) in which the second material is provided, which forms the second region.

4. The attachment according to any one of the preceding claims, **characterised in that** the at least one depression (87) is of an annular design.

5. The attachment according to claim 1, **characterised in that** the segments (17) and the openings (19) taper towards the free space (7).

6. The attachment according to claim 1 or 5, **characterised in that** at least one of the segments (17) includes at least one annularly formed cavity (57), which preferably contains the second material, wherein the at least one segment (17) comprises a circular arc-shaped wall section (59), which delimits the at least one cavity (57) opposite the free space (7), and wall regions (61) emanating from the ends of the wall section (59), which wall regions (61) laterally delimit the at least one cavity (57), wherein the wall section (59) and the wall regions (61) are formed to be resilient so that with a force acting from the free space (7) on the wall section (59), the wall regions are forced laterally outward, and thus the widths measured in the circumferential direction of the laterally adjacent openings (19) are reduced.

7. The attachment according to claim 1, **characterised in that** the at least one segment (17) comprises an inner surface (31) facing the free space (7), which preferably comprises two lateral regions (35, 37) adjacent to a central region (33).

8. The attachment according to any one of the claims 1 and claims 5 to 7, **characterised in that** the at least one segment (17) comprises an upper side (47), and in a region of the upper side (47) facing the free space, a slope (49) is provided on which a wedge (51) is placed.

9. The attachment according to claim 1, **characterised in that** the first material contains polycarbonates, polysulfones, or polypropylenes or is comprised only of these plastics, and/or wherein the second material contains soft plastics, in particular thermoplastic elastomers such as TPE, PTFE, soft PVC, or silicone, or is completely composed of these materials.

10. A syringe, carpule or the like comprising a distal end (91) and a terminal extension (75) provided thereon, which has an outer surface (79), a free end (85), and at least one depression (87) introduced at a distance thereto into the outer surface, **characterised in** an attachment (1) according to one of claims 1 to 9.

## Revendications

1. Embout pour une seringue, une cartouche (81) ou similaire, comportant :
- une extrémité distale (91) et sur celle-ci
- un prolongement terminal (79), dans lequel
- le prolongement (79) comporte une surface extérieure (83), une extrémité libre (85) et au moins une dépression (87) disposée à distance de cette dernière dans la surface extérieure, dans lequel
- l'embout (1) comporte un corps principal (5) circulaire et peut être placé sur le prolongement (79), dans lequel
- le corps principal (5) entoure un espace libre (7), le prolongement (79) pouvant être introduit dans celui-ci, dans lequel
- le corps principal (5) comprend deux matériaux, desquels un premier matériau est indéformable et un deuxième matériau est plus mou que le premier matériau indéformable et est déformable, dans lequel
- le corps principal (5) comporte au moins une région de prise, qui entre en prise dans l'au moins une dépression (87) dans l'état branché sur le prolongement (79), dans lequel
- le corps principal (5) comporte le long d'une ligne périphérique imaginaire des premières régions du premier matériau et des deuxièmes régions du deuxième matériau, dans lequel
- le deuxième matériau est formé dans l'au moins une dépression (87) dans l'état branché sur le prolongement (79), et dans lequel
- le deuxième matériau est incrusté dans le premier matériau,
**caractérisé en ce que**
- le corps principal (5) comporte un corps circulaire (15) à une extrémité (39), lequel entoure une zone circulaire du prolongement (79) dans l'état branché sur le prolongement (79), dans lequel l'au moins une dépression (87) est agencée dans cette zone du prolongement (79), et **en ce que**
- le corps circulaire (15) comporte dans la direction du pourtour, le long de la ligne périphérique imaginaire, des segments (17) formant les premières régions et des cavités (19) dans les espaces intermédiaires entre les segments, accueillant le deuxième matériau et formant les deuxièmes régions, de préférence agencés à la même distance les uns des autres, dans lequel
- dans l'état monté sur le prolongement (79), la ligne périphérique imaginaire se trouve dans la région de l'au moins une dépression (87) du prolongement (79).

2. Embout selon la revendication 1, **caractérisé en ce que** le corps principal (5) peut être obtenu par utilisation du premier matériau et du deuxième matériau dans un moulage par injection à deux composants.

3. Embout selon la revendication 1 ou 2, **caractérisé en ce que** le corps principal (5) comporte le premier matériau et comprend dans une région périphérique au moins une cavité (19), le deuxième matériau étant prévu dans celle-ci, ce qui forme la deuxième région.

4. Embout selon l'une des revendications précédentes, **caractérisé en ce que** l'au moins une dépression (87) est réalisée de manière circulaire.

5. Embout selon la revendication 1, **caractérisé en ce que** les segments (17) et les cavités (19) se rétrécissent dans la direction de l'espace libre (7).

6. Embout selon la revendication 1 ou 5, **caractérisé en ce que** l'au moins un segment (17) comporte au moins un espace creux (57) conçu de préférence de manière circulaire, qui contient de préférence le deuxième matériau, dans lequel l'au moins un segment (17) comprend une section de paroi (59) en arc de cercle, laquelle délimite l'au moins un espace creux (57) par rapport à l'espace libre (7), et aux extrémités de la section de paroi (59) se trouvent des régions de paroi (61) provenant de cette section, lesquelles délimitent latéralement au moins un espace creux (57), dans lequel la section de paroi (59) et les régions de paroi (61) sont conçus de manière élastique, de sorte que lorsqu'une force est exercée de l'espace libre (7) sur la section de paroi (59), les régions de parois (61) sont poussées latéralement vers l'extérieur et ainsi réduise la largeur, mesurée dans la direction du pourtour, des cavités (19) délimitées latéralement.

7. Embout selon la revendication 1, **caractérisé en ce que** l'au moins un segment (17) comporte une surface intérieure (31) tournée vers l'espace libre (7), laquelle comprend de préférence deux régions latérales (35, 37) jouxtant une région médiane (33).

8. Embout selon l'une des revendications 1 et 5 à 7 ci-dessus, **caractérisé en ce que** l'au moins un segment (17) comporte une face supérieure (47) et **en ce qu'**un chanfrein (49) est prévu dans une région de la face supérieure (47) tournée vers l'espace libre, sur lequel est disposé une cale (51).

9. Embout selon la revendication 1, **caractérisé en ce que** le premier matériau comprend des polycarbonates, des polysulfones ou du polypropylène ou ne consiste qu'en ces plastiques et/ou **en ce que** le second matériau comprend un plastique souple, en particulier un élastomère thermoplastique comme le TPE, le PTFE, le PVC plastifié ou la silicone ou consiste entièrement en ces matériaux.

10. Seringue, cartouche ou similaire dotée d'une extrémité distale (91) et d'un prolongement terminal (75) prévu sur celle-ci, celui-ci comportant une surface extérieure (79), une extrémité libre (85) et au moins une dépression (87) disposée à distance de cette dernière, **caractérisée par** un embout (1) selon l'une des revendications 1 à 9.
